# EUROPÄISCHE PATENTANMELDUNG

(11) **EP 1 878 422 A2**
(43) Veröffentlichungstag der Anmeldung: **16.01.2008**
(21) Anmeldenummer: 07110943.3
(22) Anmeldetag: 25.06.2007
(51) Int. Cl.: A61K 8/64, A61Q 5/00, A61Q 19/10

(54) **Tensidhaltige kosmetische Zubereitungen mit besonderen Proteinhydrolysaten**

(30) Priorität: 13.07.2006 DE 102006032666
(71) Anmelder: Beiersdorf AG, 20253 Hamburg (DE)
(72) Erfinder: Argembaux, Horst, 21465 Wentorf (DE); Bluck, Manuela, 21259 Kröppelshagen-Fahrendorf (DE); Demitz, Michael, 22529 Hamburg (DE); Köhler, Manuela, 20144 Hamburg (DE); Küther, Jörg, 25469 Halstenbek (DE); Liste, Kathrin, 88131 Lindau (DE)

(57) **Zusammenfassung**

Kosmetische Zubereitung enthaltend
a) Proteinhydrolysate aus Kaschmir-Wolle,
b) mindestens ein kationisches Tensid.

## Beschreibung

Die Erfindung betrifft kosmetische Zubereitungen, insbesondere Duschzubereitungen, Haarreinigungszubereitungen oder Haarpflegemittel, enthaltend Proteinhydrolysate aus Kaschmir-Wolle und Tenside. Die Zubereitungen sind mild zu Haut und Haaren und führen zu einer Verbesserung der Haarstruktur als auch der physikalisch-optischen Haareigenschaften.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem so genannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Wesentliche Einflüsse für den Qualitätsverlust eines Haares während seiner Alterung sind der Einfluss des Sonnenlichts, mechanische Belastungen durch intensives Kämmen oder Bürsten, aber auch Haarbehandlungen, wie Haarfärbungen und insbesondere Blondierungen sowie Haarverformungen, beispielsweise Dauerwellverfahren. Besonders oxidative Belastungen führen demnach häufig zu einer Schädigung des Haares.

Sowohl UV-A- als auch UV-B-Strahlung haben einen schädigenden Einfluss auf das Haar, der sich beispielsweise darin äußert, dass bestimmte Aminosäuren wie Cystin und Methionin abgebaut oder Schwefel-Schwefel-Bindungen des Keratins gespalten werden, was im schlimmsten Fall eine Zerstörung des Haars zur Folge haben kann. Weiterhin stellen Haar und Kopfhaut Teile des Körpers dar, die aufgrund ihrer Position beim Aufenthalt im Freien einer erheblichen Menge an UV-Strahlung ausgesetzt sind.

Ein Ziel der Haarpflege und damit auch der vorliegenden Erfindung ist es, den Naturzustand des frisch nachgewachsenen Haares über einen möglichst langen Zeitraum zu erhalten und im Fall eines Verlusts wieder herzustellen. Seidiger Glanz, geringe Porosität und ein angenehmes, glattes Gefühl gelten als Merkmale für natürliches, gesundes Haar.

Seit Ende des vergangenen Jahrhunderts werden Produkte zur Haarpflege gezielt entwickelt. Dies führte zu einer Vielzahl von Präparaten sowohl für die allgemeine Haarpflege als auch zur Behebung von Anomalien des Haares und der Kopfhaut. Im allgemeinen werden heutzutage Haarpflegekosmetika verwendet, welche entweder dazu bestimmt sind, nach dem Einwirken aus dem Haar wieder ausgespült zu werden, oder welche auf dem Haar verbleiben sollen. Letztere können so formuliert werden, dass sie nicht nur der Pflege des einzelnen Haars dienen, sondern auch das Aussehen einer Frisur insgesamt verbessern. Solcherart gepflegtes Haar zeichnet sich durch einen angenehmen Griff, natürlichen Glanz, vermehrte Fülle, Geschmeidigkeit und somit gute Frisierbarkeit und Festigkeit und somit gutem Frisurensitz aus.

Produkte die ausschließlich der Pflege des Haares dienen, werden allgemein als Haarkonditioniermittel oder Conditioner bezeichnet. Diese können nach einer mehr oder weniger langen Verweilzeit auf dem Haar ausgespült werden (Rinse-off Produkte, z. B. Spülungen, Haar-Kuren) oder sie verbleiben nach der Anwendung auf dem Haar (Leave-on Produkte). Die Produkte können verschiedene Konsistenzen haben, so dass sie ganz unterschiedlich appliziert werden können. Es können Emulsionen oder Gele sein oder dünnflüssige Lösungen, die z. B. über Sprühapplikationen aufgebracht werden, oder Schäume, die z. B. durch geeignete Druckgaspackungen oder spezielle Schaumpumpen bei der Applikation erzeugt werden. Cremige, trübe und klar transparente Produkte sind im Markt zu finden.

Je nach Verwendungszweck findet man ganz unterschiedliche Wirkstoffe oder Kombinationen von Wirkstoffen in solchen Conditionern. Manche, die eher dem Schutz des Haares dienen, wie Antioxidantien oder UV-Filter, andere die das Haar geschmeidig machen wie z. B. kationische Tenside. Eine immer größere Bedeutung bekommen polymere Wirkstoffe, die je nach Art, Molgewicht und Ladung ganz unterschiedliche Eigenschaften haben. Im Vordergrund steht dabei jedoch eindeutig eine Verbesserung der Oberflächenbeschaffenheit des Haares.

Trotz einer großen Vielfalt von Produkten, die dem Verbraucher zur Verfügung stehen, sind einige Nachteile noch nicht restlos beseitigt. So zeigen Polymere, die dem Haar eher eine gewisse Festigkeit und somit Volumen geben häufig ein schlechtes Griffempfinden und eine schlechte Kämmbarkeit; Polymere, die das Haar geschmeidig machen führen häufig zu einer Beschwerung, was mit mangelndem Volumen verbunden ist. Auch der Einsatz von Stärkederivaten konnte in der Vergangenheit den geschilderten Nachteilen des Standes der Technik nicht abhelfen.

Der Stand der Technik lässt es an Haarpflegeformulierungen mangeln, welche dem geschädigten Haar in befriedigender Weise Pflege zukommen ließen. Aufgabe ist es daher, auch diesen Nachteilen des Standes der Technik Abhilfe zu schaffen.

US 4751074 offenbart Haarspülungen mit kationischem Tensid und besonders modifizertem Keratinhydrolysat.

US 4436722 offenbart Zubereitungen mit Oxidationsprodukten der Keratins.
DE 10354316 offenbart den Einsatz von Proteinhydrolysaten unter anderem auch aus Kaschmir-Wolle.

Die herkömmlichen Proteinhydrolysate haben eine stark ausgeprägte Neigung zu Verfärbungen sowohl bei Einarbeitung in eine Formulierung als auch bei anschließenden Lagerung. Des Weiteren kommt es hier besonders häufig zu negativen geruchlichen Veränderungen des Rohstoffes, der auch die Gesamtformulierung negativ beeinflussen kann. Hierdurch müssen bei einem Einsatz dieser Proteinhydrolysate die tensidhaltigen Formulierungen mit entsprechenden Zusatzstoffen wie Stabilisatoren oder einer erhöhten Parfümkonzentration versehen werden.

Ziel der vorliegenden Erfindung ist es auch Formulierungsvarianten zu schaffen, die verbesserte sensorische und taktile Eigenschaften aufweisen als Zubereitungen des Standes der Technik. Insbesondere gilt es auch Zubereitungen zur Verfügung zu stellen, deren Biopolymerbestandteile in deutlichem Maße aus den Formulierungen zur Haar-, Körper- und Kopfbodenpflege freigesetzt werden können.

Die Aufgabe der vorliegenden Erfindung ist auch eine kosmetische Reinigungs- und Pflegezubereitung bereit zu stellen, die sich insbesondere durch gute Haut- und Haarverträglichkeit, gutes Schaumverhalten und angenehme Haarpflege und -frisieren auszeichnen.

Gelöst wird dieses Bündel an Aufgaben durch eine kosmetische Zubereitung enthaltend Proteinhydrolysate aus Kaschmir-Wolle und mindestens ein kationisches Tensid. Dabei ist es bevorzugt, wenn das Proteinhydrolysat aus Kaschmirwolle ein Gewichtsmittel des Molekulargewichtes von 5000 bis 8000, besonders bevorzugt 6500 bis 7500 hat. Weiter ist es bevorzugt, wenn der Gehalt an Proteinhydrolysat 0,001 bis 5 Gew.-% beträgt, besonders bevorzugt 0,01 bis 2 Gew.-%, ganz besonders bevorzugt 0,05 bis 1,5 Gew.-%. Dabei ist es bevorzugt, wenn als kationisches Tensid 1 bis 10 Gew.-% quaternäre Ammoniumsalze enthalten sind. Weiterhin ist es besonders bevorzugt, wenn ein Fettalkohol von 0,1 bis 10 Gew.-% enthalten ist. Besonders bevorzugt ist es, wenn ein weiteres Tensid aus der Gruppe der anionischen, amphoteren oder nicht ionischen Tenside in Konzentrationen von 0,1 bis 20 Gew.-%, bevorzugt 3 bis 10 Gew.-%, besonders bevorzugt 4 bis 7 Gew.-% enthalten ist. Weiterhin ist es bevorzugt, wenn ein ethoxylierter Fettalkohol mit 2 bis 75 Ethylenoxyeinheiten, bevorzugt bis 39 Ethylenoxyeinheiten, besonders bevorzugt bis 5 Ethylenoxyeinheiten in Konzentrationen von 0,1 bis 2 Gew. % enthalten ist. Gleichfalls ist es bevorzugt, wenn die Zubereitung eine Emulsion mit weniger als 10 Gew. % Emulgator darstellt. Besonders bevorzugt ist es, wenn die Zubereitung Crotein Cashmere oder Cashmilan LS 9604 enthält. Die Erfindung umfasst auch die Verwendung einer solchen Zubereitung zur topischen Anwendung auf Haut und/oder Haaren, die Verwendung einer solchen Zubereitung als Duschzubereitung, Haarpflegezubereitung, Shampoo oder Haarpflegemittel zur Anwendung an Mensch oder Tier sowie die Verwendung einer solchen Zubereitung zur stabilen Bereitstellung von erfindungsgemäßen Proteinhydrolysaten in Kosmetikprodukten.

Die Erfindung umfasst darüber hinaus auch die Verwendung der Zubereitungen auf der Haut und/oder dem Haar. Für den Fachmann überraschend wurde festegestellt, dass Extrakte von Ziegenhaar, hier insbesondere die der Kaschmirziege, bisher in kosmetischen Formulierungen, hier insbesondere tensidhaltige Formulierungen, noch nicht zum Einsatz gekommen sind. Aufgrund der haar- und hauteigenen Bestandteile ist der Stoff in der Lage sowohl die Haut als auch die Haare zu schützen.

Bei Haarpflegeformulierungen ist darüber hinaus ein positiver Einfluss auf die Sensorik zu spüren, der sich in einem angenehmeren Griff äußert.

Aufgrund des natürlichen Ursprungs dieses Rohstoffes zeigt dieser eine gute biologische Abbaubarkeit und eine gute Hautverträglichkeit.

Die Aufgabe umfasst weiterhin die stabile Einarbeitung dieses oxidationsempfindlichen Gemisches. Extrakte aus Haaren der Kaschmirziege lassen sich überraschenderweise besonders gut und stabil in tensidhaltige Produkte einarbeiten, ohne dass ein weiterer Einsatz von so genannten Hilfsstoffen zur Stabilisierung unbedingt erforderlich ist.

Kationische Filmbildner, eingearbeitet in Haarreinigungsprodukte, zeigen bei zu häufiger Anwendung so genannte "Build up"-Effekte. Darunter versteht man die Anlagerung dieser Rohstoffe auf dem Haar, wodurch dieses schwer und stumpf wird. Die Proteinhydrolysate aus Haaren der Kaschmirziege zeigen aufgrund ihrer haareigenen Bestandteile diese Art "Build-up"Effekte nicht.

Gegenüber kationischen Filmbildner zeigt das Proteinhydrolysat aus Haaren der Kaschmirziege gute Stabilitätsergebnis beim Einsatz mit anionischen Trübungsmitteln. Diese Trübungsmitteln neigen hingegen bei Kombination mit herkömmlichen kationischen Filmbildern vermehrt dazu in der Formulierung aufzurahmen oder sich abzusetzen.

### Methode der Kämmkraftmessung (nass):

Bei der Messung Der Kämmkraft werden die Kräfte erhoben, die benötigt werden, um das entsprechende Haarmaterial im nassen Zustand durchzukämmen.

Die Untersuchung erfolgt in zwei Schritten: Im ersten Schritt werden die Kämmkräfte der unbehandelten Haarproben (Nullwerte, 5 Strähnen mildgebleicht, 10 Messungen pro Strähne) erhoben. Im zweiten Schritt erfolgt nach der Produktapplikation die erneute Bestimmung der mittleren Kräfte. Bei der Auswertung werden die relativen Änderungen miteinander verglichen

Die erfindungsgemäßen Kombinationen regen den Stoffwechsel in der Kopfhaut an und fördert die Feuchtigkeitsanreicherung auf der Kopfhaut und im Haar und zeichnen sich vor allem durch gute Haut- und Haarverträglichkeit aus. Ferner liefern die Aminosäurebestandteile die Faktoren an Haut und Haar zurück, welche wie "Magnete" für die ideale Feuchtigkeit in der Haut und seinen Anhangsgebilden führen. Das wirkt sich insbesondere positiv auf die Frisierbarkeit des Haares aus, welches durch zu wenig oder zu schnell flüchtige Feuchtigkeit seine Frisierbarkeit durch elektrostatische Aufladung zu verlieren droht.

Durch die topische Anwendung der Zubereitung auf der Haut und/oder dem Haar werden insbesondere folgende positive Eigenschaften des behandelten Haares bzw. Haut festgestellt:
- Verbesserung der Frisierbarkeit des Haares
- Vermeidung des negativ Effektes der statischen Aufladung der Haare
- stabilisierenden Effekt auf die Haarstruktur
- Schutz und Pflege der Haut
- pflegend für Haar und Kopfhaut, insbesondere über einen längeren Zeitraum nach der Anwendung
- strukturglättende Wirkung auf Haut und Haar
- Verleiht dem Haar natürlichen Schimmer und attraktives Volumen
- das Haar ist leichter zu kämmen und einfacher zu stylen
- schützt das Haar und die Kopfhaut beim Fönen
- schützt vor elektrostatischer Aufladung durch Balancierung und Ausgleich des Feuchtigkeitshaushaltes der Haare

Bevorzugt werden die erfindungsgemäßem Zubereitungen silikonfrei hergestellt und vertrieben.

Auch als Konditioner zeigten sich überraschend positive Eigenschaften, so wirkt das mit den erfindungsgemäßen Zubereitungen behandelte Haar äußerst gepflegt und lässt sich gut frisieren. Dies ist beispielsweise an der Kämmbarkeit, des Anscheins und der Fülle des Haares erkennbar.

Proteinhydrolisate der Kaschmir-Wolle sind bereits beschrieben. Sie werden beispielsweise von Laboratoires Serobiologiques, Frankreich, hergestellt und vertrieben und als "Cashmilan LS 9604" vertrieben..

Ein zusätzlicher Gehalt an Antioxidantien ist in kosmetischen und/oder dermatologischen Zubereitungen im allgemeinen bevorzugt.

Die Mittel gemäß der Erfindung können beispielsweise als aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels.

Als Treibmittel für aus Aerosolbehältern versprühbare kosmetische oder dermatologische Zubereitungen im Sinne der vorliegenden Erfindung, sind die üblichen bekannten leichtflüchtigen, verflüssigten Treibmittel, beispielsweise Dimethylether, Kohlenwasserstoffe (Propan, Butan, Isobutan) geeignet, die allein oder in Mischung miteinander eingesetzt werden können. Auch Druckluft, Stickstoff, Stickstoffdioxid oder Kohlendioxid oder Gemische aus diesen Substanzen sind vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Es kann auch von Vorteil sein, erfindungsgemäße Zubereitungen mit UV-A-Filtern zu versetzen, die bisher üblicherweise in kosmetischen Zubereitungen enthalten sind. Es können die für die UV-B-Kombination verwendeten Mengen eingesetzt werden.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Der pH-Wert der Zubereitungen kann in bekannter Weise durch Zugabe von Säuren oder Basen eingestellt werden, vorzugsweise durch Zugabe von Puffergemischen, z. B. auf Basis von Citronensäure/Citrat oder Phosphorsäure Phosphat-Puffergemischen. Vorzugsweise liegt der pH-Wert unter 10, z. B. im Bereich von 2 - 7, insbesondere im Bereich von 3 - 5.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

### Beispiel Haarkuren:

| | **1** | **2** | **3** | **4** | **5** | **5** |
|---|---|---|---|---|---|---|
| Hydroxypropylmethylcellulose | 0,5% | 0,5% | 0,5% | 0,3% | 0,4% | 0,5% |
| Cetrimoniumbromid | 1,0 | - | 0,8 | - | 0,5 | 0,7 |
| Behentrimoniumchlorid | - | 0,7 | 0,3 | - | - | - |
| Distearoylethyl Hydroxyethylmonium Methosulfate | - | - | - | 1,2 | - | 0,7 |
| Palmitamidopropyltrimonium Chlorid | - | - | - | - | 0,5 | - |
| Glycerin | 3,0 | 3,0 | 3,0 | 2,5 | 2,0 | - |
| Cetearylalkohol | 2,5 | 2,5 | 2,5 | 2,5 | 2,0 | 2,5 |
| Glycerylstearat | 2,0 | 2,0 | 2,0 | 2,0 | 2,0 | 1,8 |
| Polyquaternium-10 | 0,1 | - | - | - | 0,1 | - |
| Guar Hydroxypropyl Trimonium - Chlorid | | 0,2 | - | 0,1 | - | - |
| Proteinhydrolysat | 0,1 | 0,2 | 0,2 | 0,4 | 0,1 | 0,3 |
| Konservierungsmittel, Parfüm, pH-Einstellung und Lösungsvermittler | q.s. | q.s. | q.s. | q.s. | q.s. | q.s. |
| Wasser | ad 100 | ad 100 | ad 100 | ad 100 | ad 100 | ad 100 |

### Beispiel Haarspülungen:

| | **1** | **2** | **3** | **4** | **5** | **6** |
|---|---|---|---|---|---|---|
| Cetrimoniumchlorid | 1,0 | 0,5 | 0,5 | - | 0,5 | 0,5 |
| Distearoylethyl Hydroxyethylmonium Methosulfate | - | - | - | 1,0 | - | 0,5 |
| Palmitamidopropyltrimonium Chlorid - | | - | - | - | 0,8 | - |
| Behentrimoniumchlorid | - | 0,2% | 0,3 | - | - | - |
| Cetearylalkohol | 3,0 | 2,5 | 2,8 | 3,0 | 2,6 | 2,8 |
| Glycerin | 3,0 | 3,0 | 3,0 | 2,8 | 2,5 | 2,0 |
| Hydroxyethylcellulose | 0,2 | 0,2 | 0,2 | 0,2 | 0,2 | 0,2 |
| Polyquaternium-10 | 0,1 | - | - | - | - | 0,1 |
| Guar Hydroxypropyl Trimonium Chlorid | - | 0,2 | - | - | - | - |
| Proteinhydrolysat | 0,2 | 0,1 | 0,05 | 0,2 | 0,1 | 0,3 |
| Konservierungsmittel, Parfüm, pH-Einstellung und Lösungsvermittler | q.s. | q.s. | q.s. | q.s. | q.s. | q.s. |
| Wasser | ad 100 | ad 100 | ad 100 | ad 100 | ad 100 | ad 100 |

### Beispiel Spray-Conditioner:

| | **1** | **2** | **3** | **4** | **5** | **6** | **7** |
|---|---|---|---|---|---|---|---|
| Cetrimoniumchlorid | 0,2 | 0,1 | 0,8 | 0,5 | - | 0,2 | - |
| Behentrimoniumchlorid | - | 0,2 | 0,3 | - | - | - | - |
| Distearoylethyl Hydroxyethylmonium Methosulfate | - | - | - | - | 0,2 | - | 0,2 |
| Palmitamidopropyltrimonium Chlorid | - | - | - | - | - | 0,1 | 0,1 |
| Benzophenone-4 | 0,05 | 0,03 | - | - | - | - | 0,03 |
| PVP/VA Copolymer | - | 0,7 | - | - | - | 0,2 | - |
| Polyquaternium-10 | 0,1 | - | - | - | 0,1 | - | - |
| Polyquaternium-4 | 0,2 | - | - | - | - | 0,2 | 0,2 |
| Propylene Glycol | - | - | - | 3,0 | 2,0 | - | 2,0 |
| Polyquaternium-11 | - | - | 0,2 | - | - | - | - |
| Panthenol | 0,1 | - | 0,2 | 0,1 | - | 0,2 | - |
| Glyceryllsostearate | - | - | - | 0,4 | - | - | - |
| Isoceteth-20 | - | - | - | 0,8 | - | - | - |
| Dicaprylyl Carbonate | - | - | - | 0,5 | - | - | - |
| Proteinhydrolysat | 0,2 | 0,05 | 0,1 | 0,1 | 0,3 | 0,5 | 0,2 |
| Konservierungsmittel, Parfüm, pH-Einstellung und Lösungsvermittler | q.s. | q.s. | q.s. | q.s. | q.s. | q.s. | q.s. |
| Wasser | ad 100 | ad 100 | ad 100 | ad 100 | ad 100 | ad 100 | ad 100 |

### Beispiel Leave-on Conditioner:

| | **1** | **2** | **3** | **4** | **5** | **6** | **7** |
|---|---|---|---|---|---|---|---|
| Cetylalcohol | 1,5 | 1,8 | 2,0 | - | 1,0 | 1,7 | 2,0 |
| Cetrimoniumchlorid | 0,3 | 0,1 | 0,5 | 0,5 | - | 0,2 | - |
| Behentrimoniumchlorid | - | 0,2 | - | - | - | - | 0,2 |
| Distearoylethyl Hydroxyethylmonium Methosulfate | - | - | - | - | 0,5 | - | 0,2 |
| Palmitamidopropyltrimonium Chlorid | - | - | - | - | - | 0,2 | - |
| Benzophenone-4 | 0,05 | 0,03 | - | 0,1 | - | 0,1 | - |
| PVP/VA Copolymer | 0,4 | - | - | - | 0,3 | - | - |
| Polyquaternium-37 | - | - | - | 1,0 | - | - | - |
| Polyquaternium-4 | - | - | - | 0,2 | - | 0,1 | - |
| Polyquaternium-10 | - | - | 0,5 | - | 0,3 | - | 0,2 |
| Panthenol | 0,1 | - | 0,2 | 0,1 | - | - | 0,2 |
| Hydroxyethylcellulose | - | - | - | 0,3 | - | - | 0,2 |
| Acrylates/C10-30 Alkyl Acrylates Crosspolymer | 0,5 | 0,3 | 0,2 | - | - | - | - |
| C12-13 Alkyl Lactate | 2,0 | 1,0 | 1,5 | 1,0 | 1,5 | 1,0 | 1,0 |
| Laureth-4 | - | - | - | 0,5 | - | - | - |
| Aluminium Starch Octenylsuccinate - | | - | - | 1,0 | - | - | - |
| Dicaprylyl Carbonate | - | - | - | 1,0 | - | - | - |
| Proteinhydrolysat | 0,2 | 0,1 | 0,1 | 0,1 | 0,4 | 0,3 | 0,1 |
| Konservierungsmittel, Parfüm, pH-Einstellung und Lösungsvermittler | q.s. | q.s. | q.s. | q.s. | q.s | q.s | q.s |
| Wasser | ad 100 | ad 100 | ad 100 | ad 100 | ad 100 | ad 100 | ad 100 |

## Patentansprüche

1. Kosmetische Zubereitung enthaltend
a) Proteinhydrolysate aus Kaschmir-Wolle,
b) mindestens ein kationisches Tensid.

2. Zubereitung nach Anspruch 1 wobei das Proteinhydrolysat aus Kaschmirwolle ein Gewichtsmittel des Molekulargewichtes von 5000 bis 8000, besonders bevorzugt 6500 bis 7500 hat.

3. Zubereitung nach Anspruch 1 wobei der Gehalt an Proteinhydrolysat 0,001 bis 5 Gew.-% beträgt, bevorzugt 0,01 bis 2 Gew.-%, besonders bevorzugt 0,05 bis 1,5 Gew.-%.

4. Zubereitung nach einem der vorangehenden Patentansprüche **dadurch gekennzeichnet, dass** als kationisches Tensid 1 bis 10 Gew.-% quaternäre Ammoniusalze enthalten sind.

5. Zubereitung nach einem der vorangehenden Patentansprüche **dadurch gekennzeichnet, dass** ein Fettalkohol von 0.1 bis 10 Gew. % enthalten ist.

6. Zubereitung nach einem der vorangehenden Patentansprüche **dadurch gekennzeichnet, dass** ein weiteres Tensid aus der Gruppe der anionischen, amphoteren oder nicht ionischen Tenside in Konzentrationen von 0,1 bis 20 Gew.-%, bevorzugt 3 bis 10 Gew.-%, besonders bevorzugt 4 bis 7 Gew.-% enthalten ist.

7. Zubereitung nach einem der vorangehenden Patentansprüche **dadurch gekennzeichnet, dass** ein ethoxylierter Fettalkohol mit 2 bis 75 Ethylenoxyeinheiten, bevorzugt bis 39 Ethylenoxyeinheiten, besonders bevorzugt bis 5 Ethylenoxyeinheiten in Konzentrationen von 0,1 bis 2 Gew.-% enthalten ist.

8. Zubereitung nach einem der vorangehenden Patentansprüche **dadurch gekennzeichnet, dass** die Zubereitung eine Emulsion mit weniger als 10 Gew.-% Emulgator darstellt.

9. Zubereitung nach einem der **dadurch gekennzeichnet, dass** die Zubereitung Crotein Cashmere oder Cashmilan LS 9604 enthält.

10. Verwendung der Zubereitung nach einem der vorangehenden Patentansprüche zur topischen Anwendung auf Haut und/oder Haaren.

11. Verwendung der Zubereitung nach einem der vorangehenden Patentansprüche als Duschzubereitung, Haarpflegezubereitung, Shampoo oder Haarpflegemittel zur Anwendung an Mensch oder Tier.

12. Verwendung der Zubereitung nach einem der vorangehenden Patentansprüche zur stabilen Bereitstellung von erfindungsgemäßen Proteinhydrolysaten in Haarkosmetikprodukten.
